# EUROPEAN PATENT APPLICATION

(11) **EP 4 520 350 A1**
(43) Date of publication of application: **12.03.2025**
(21) Application number: 23799714.3
(22) Date of filing: 04.05.2023
(51) Int. Cl.: A61K 47/42, A61K 47/64, A61K 47/68, A61K 38/26, A61P 25/00

(54) **FUSION PROTEIN COMPRISING GLP-1, IMMUNOGLOBULIN FC, AND IGF-1 AND USE THEREOF**

(30) Priority: 04.05.2022 KR 20220055161
(71) Applicant: ImmunoForge Co., Ltd., Gasan digital 1-ro, Geumcheon-gu Seoul 08591 (KR)
(72) Inventor: KIM, Jong-Won, Seoul 08325 (KR); CHOI, Hyo-Jung, Incheon 21997 (KR); CHANG, Kiho, Incheon 22002 (KR); AHN, Sung-Min, Seoul 06581 (KR)
(74) Representative: Icosa
(86) International application number: PCT/KR2023/006139
(87) International publication number: WO 2023/214833

(57) **Abstract**

The present invention relates to a fusion protein comprising GLP-1, immunoglobulin Fc region, and IGF-1, and use thereof. The fusion protein of the present invention has excellent efficiency in blood-brain barrier permeability, and thus can be widely used for the effective treatment of neurological diseases.

## Description

### [Technical Field]

The present invention relates to a fusion protein containing a GLP-1, an immunoglobulin Fc region, and an IGF-1, and use thereof.

### [Background Art]

Blood-brain barrier (BBB) is a cellular barrier composed of tight junctions with a high degree of electrical resistance of at least 0.1 Ω·m between vascular endothelial cells that are adjacent to related pericytes and astrocytes. The BBB acts as a barrier with highly selective permeability to separate the circulating blood from brain extracellular fluids in the central nervous system (CNS), thereby playing the role of a gateway to protect the central nervous system by regulating the entry and exit of nutrients and other substances with respect to the brain.

Usually, the blood-brain barrier allows not only the selective transfer of molecules, such as glucose and amino acids that are essential for brain functions, but also the passage of water, some kinds of gases, and fat-soluble molecules via passive diffusion. However, the blood-brain barrier blocks the entry and exit of lipophilic, potential neurotoxins through an active transport mechanism mediated by permeable p-glycoprotein. As a result, neurological disease drugs, such as drugs with large molecular weights or low-molecular-weight drugs with low brain penetration that need to act inside the brain, cannot penetrate the blood-brain barrier.

Therefore, the blood-brain barrier serves to block the transport of bacteria, pathogens, and potential hazardous substances in the blood to the brain that can be carried through the blood, but due to such a vascular barrier, most central nervous system drugs show low efficiency of transcranial delivery, and in order to compensate for this, such drugs are administered at a high dose, which may cause serious side effects in surrounding organs. Consequently, despite continuous research (KR 10-2211721), the discovery of an efficient drug delivery system capable of penetrating the blood-brain barrier is required to prevent negative systemic effects and ensure therapeutic effects of chemo-drugs.

### [Disclosure]

### [Technical Problem]

The present invention developed a fusion protein capable of penetrating the blood-brain barrier, exhibiting sustained efficacy upon drug administration, and showing an effect of preventing and delaying the progression of a neurological disease, and thus completed the present invention.

### [Technical Solution]

In accordance with an aspect of the present invention, there is provided a fusion protein containing a GLP-1, an immunoglobulin Fc region, and an IGF-1.

In accordance with another aspect of the present invention, there is provided a pharmaceutical composition for the prevention or treatment of a neurological disease, the pharmaceutical composition containing as an active ingredient a fusion protein containing a GLP-1, an immunoglobulin Fc region, and an IGF-1.

In accordance with another aspect of the present invention, there is provided a method for preventing or treating a neurological disease, the method including administering the composition to a subject.

In accordance with another aspect of the present invention, there is provided use of a fusion protein containing a GLP-1, an immunoglobulin Fc region, and an IGF-1 for preventing, alleviating, or treating a neurological disease.

In accordance with another aspect of the present invention, there is provided use of the pharmaceutical composition for preventing or treating a neurological disease.

### [Advantageous Effects]

The fusion protein of the present invention presents a novel type of candidate substance for the treatment of a neurological disease, which has an enhanced blood-brain barrier penetration efficiency, and thus can be widely utilized in the effective treatment of neurological diseases.

### [Brief Description of Drawings]

FIG. 1 shows a schematic diagram of the structure of PF1802, a fusion protein.
FIG. 2 shows a graph illustrating the results of Saos-2 proliferation assay to investigate the IGF-1 activity among PF1802 candidate substances.
FIG. 3 shows graphs confirming the IGF-1 activity of PF1802 candidate substances through IGF-1R phosphorylation assay.
FIG. 4 shows graphs confirming the GLP-1 activity of PF1802 candidate substances through cAMP assay.
FIG. 5 shows a graph illustrating the mouse PK results of PF1802 candidate substances.
FIG. 6 shows graphs illustrating the ELISA results to investigate the increase in binding affinity to human FcRn for PF1802_M020, M021, and M022 with induced blood half-life increasing Fc.
FIG. 7 shows figures confirming the mouse BBB penetration of PF1802_M008, through IVIS imaging analysis.
FIG. 8 shows graphs confirming the NMJ improvement effect of PF1802_M008 in amyotrophic lateral sclerosis (ALS) animal models.
FIG. 9 shows figures illustrating the SDS-PAGE results to investigate the structures of PF1802_M023, M024, and M025 selected through substance optimization.
FIG. 10 shows graphs illustrating the SPR results to investigate the binding affinity of PF1802_M024 and M025 selected through substance optimization.
FIG. 11 shows graphs confirming the NMJ improvement effect of PF1802_M024 in amyotrophic lateral sclerosis (ALS) animal models.
FIG. 12 shows a graph confirming the Iba1 reduction of PF1802_M024 in amyotrophic lateral sclerosis (ALS) animal models.
FIG. 13 shows figures confirming mouse BBB penetration of PF1802_M024.

### [Best Mode for Carrying Out the Invention]

The present application will be specifically described as follows. Each description and exemplary embodiment disclosed herein may also be applied to other descriptions and exemplary embodiments. That is, all combinations of various elements disclosed herein fall within the scope of the present invention. Furthermore, the scope of the present invention is not limited by the specific description below.

Furthermore, a person skilled in the art will recognize or be able to ascertain many equivalents to the specific embodiments of the present invention described herein, by using no more than routine experimentation. Furthermore, these equivalents are intended to be included in the present invention.

In accordance with an aspect of the present invention, there is provided a fusion protein containing a GLP-1, an immunoglobulin Fc region, and an IGF-1.

To achieve the aspect, a drug delivery system for blood-brain barrier (BBB) penetration containing insulin-like growth factor 1 (IGF-1) is provided.

As used herein, the term "blood-brain barrier" or "(BBB)" refers to a cellular barrier composed of tight junctions having a significantly high electrical resistance of at least 0.1 Ω between vascular endothelial cells that are adjacent to related pericytes and astrocytes, and it acts as a barrier having highly selective permeability to separate the circulating blood from brain extracellular fluids in the central nervous system (CNS), thereby playing a role of a gateway to protect the central nervous system by regulating the entry and exit of nutrients and other substances to the brain.

As used herein, the term "drug delivery system" refers to a delivery system for efficiently delivering a drug exhibiting therapeutic activity to a target tissue or organ, and the drug delivery system of the present invention can especially penetrate the blood-brain barrier to deliver a drug to the brain with high efficiency.

Specifically, the drug delivery system of the present invention includes an IGF-1, and in a more specific aspect, the drug delivery system may be in the form of a protein in which an IGF-1 and an immunoglobulin Fc region are linked, but is not limited thereto.

The drug delivery system of the present invention can exhibit therapeutic efficacy as a drug delivery system itself, as well as efficiently deliver a drug linked to the drug delivery system by penetrating the blood-brain barrier, thereby allowing the drug to exhibit therapeutic activity in the brain. In addition, the drug delivery system increases the drug's half-life, allowing the drug efficacy to last longer and reducing the number of times of administrations.

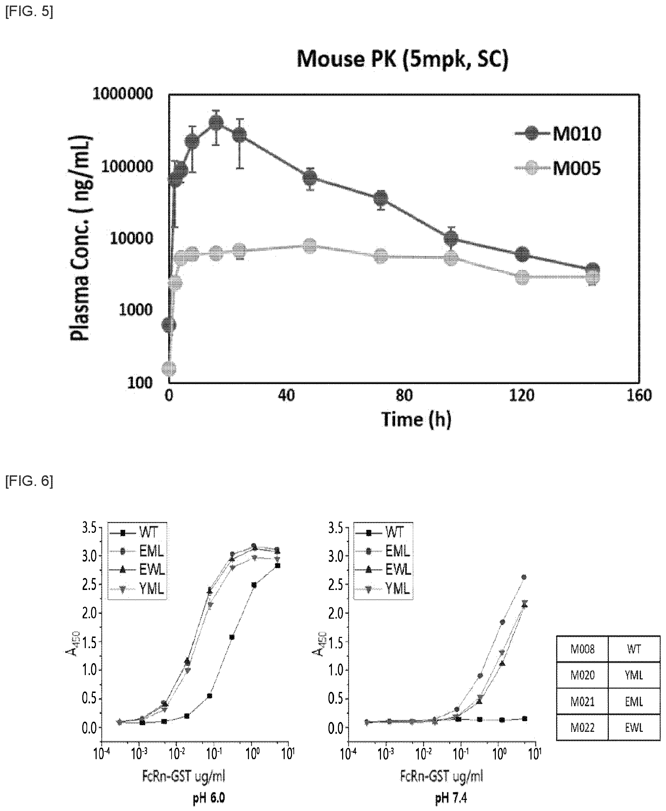

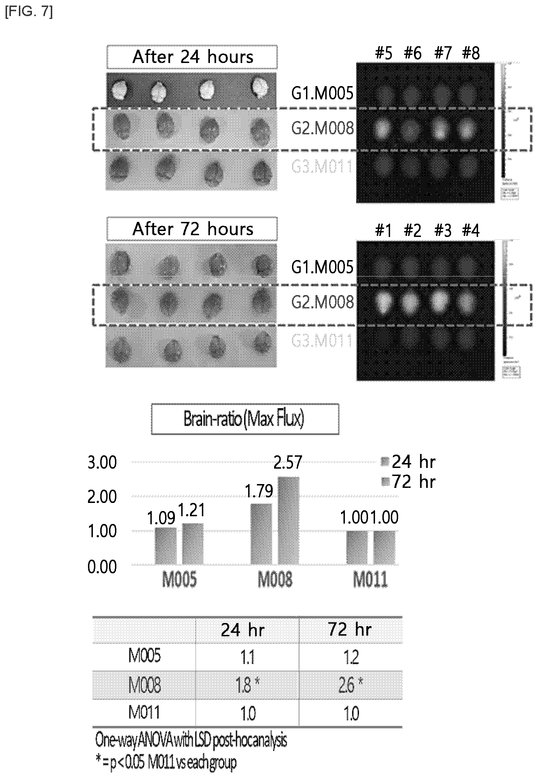

In an embodiment of the present invention, as a result of pharmacokinetic evaluation of a fusion protein containing a GLP-1, an immunoglobulin Fc region, and an IGF-1, the fusion protein increased the half-life (FIG. 5), and as a result of brain observation after the intraperitoneal administration of a fluorescently labeled substance to mice, the fluorescence intensity increased over time, indicating excellent BBB penetration (FIG. 7).

As used herein, the term "insulin-like growth factor 1" or "IGF-1" refers to a cell growth factor with a similar structure to insulin, which plays an important role in normal growth and health maintenance. IGF-1 was originated from a peptide existing in the human body. The IGF-1 of the present invention includes not only a native form but also a derivative or mutant thereof. The derivative or mutant refers to one in which at least one amino acid is mutated in the native sequence by substitution, deletion, addition, or the like, and retains its original activity. The amino acid sequence of IGF-1 is not particularly limited, but may include the amino acid sequence of SEQ ID NO: 9 or may include a sequence in which a mutation, such as a substitution, a deletion, or an addition, occurs at the amino acid corresponding to at least one of positions 3, 49, 67, 70, and a combination thereof in the amino acid sequence of SEQ ID NO: 9. Specifically, the amino acid corresponding to position 3 may be substituted with alanine, the amino acid corresponding to position 49 may be substituted with alanine, the amino acid corresponding to position 67 may be substituted with threonine, and the amino acid corresponding to position 70 may be substituted with thymine. More specifically, the amino acid sequence of SEQ ID NOS: 10, 11, or 12 may be included. The amino acid sequence of SEQ ID NO: 9, 10, 11, or 12 may include an amino acid sequence having at least 70%, 75%, 80%, 85%, 90%, 95%, 96%, 97%, 98%, 99%, 99.5%, 99.7%, or 99.9% homology or identity to the amino acid sequence set forth in SEQ ID NO: 9, 10, 11, or 12. It would be obvious that any protein that has an amino acid sequence with deletion, modification, substitution, conservative substitution, or addition in a portion thereof may also be included within the range of the present application as long as the amino acid sequence has such homology or identity and exhibits efficacy corresponding to the protein of the present application.

For example, the amino acid sequence may include an amino acid sequence having a sequence addition or deletion, a naturally occurring mutation, a silent mutation, or a conservative substitution that does not alter functions of the protein of the present application, at the N-terminus, C-terminus, and/or inside the amino acid sequence.

As used herein, the term "conservative substitution" refers to a substitution of one amino acid with another amino acid that has similar structural and/or chemical properties. The protein may have, for example, one or more conservative substitutions while still retaining one or more biological activities. Such an amino acid substitution may be generally made on the basis of similarity in polarity, charge, solubility, hydrophobicity, hydrophilicity, and/or amphipathic nature of the residues. For example, among the electrically charged amino acids, positively charged (basic) amino acids include arginine, lysine, and histidine; and negatively charged (acidic) amino acids include glutamic acid and aspartic acid. Among the uncharged amino acids, nonpolar amino acids include glycine, alanine, valine, leucine, isoleucine, methionine, phenylalanine, tryptophan, and proline; polar or hydrophilic amino acids include serine, threonine, cysteine, tyrosine, asparagine, and glutamine. Among the amino acids, aromatic amino acids include phenylalanine, tryptophan, and tyrosine.

As used herein, the term "homology" or "identity" refers to the degree of similarity between two given amino acid sequences or nucleic acid base sequences, and may be expressed as a percentage. The terms homology and identity may be often used interchangeably with each other.

The sequence homology or identity of conserved polynucleotides or proteins may be determined by standard alignment algorithms, and default gap penalties established by a program to be used may be used together. Substantially, homologous or identical sequences may hybridize under moderately or highly stringent conditions along their entire sequence or a part thereof. It would be obvious that the hybridization also includes hybridization with a polynucleotide containing usual codons or codons considering codon degeneracy in the polynucleotide.

In the present invention, the IGF-1 has functions of protecting neurons, protecting neurons against excitatory substances, and improving blood-brain barrier (BBB) permeability, thereby enhancing the preventive or therapeutic effects on neurological diseases.

For the purpose of the present invention, the IGF-1 of the present invention may include a modification in the wild-type IGF-1 amino acid sequence of SEQ ID NO: 9 for blood-brain barrier permeability. The type of modification introduced for blood-brain barrier permeability is not limited, but any modification that is introduced for the inhibition of binding ability to IGFBP may be included in the present invention without limitation. Specifically, the IGF-1 of the present invention may be a mutant including an amino acid substitution to inhibit the binding ability to IGFBP, and more specifically, a mutant including the amino acid sequence of SEQ ID NO: 10, 11, or 12.

For example, at least one amino acid substitution in the sequence of IGF-1 can lead to the inhibition of the binding to IGFBP protein, and non-limiting examples of an amino acid that may be included in the IGF-1 of the present invention may include are as follows: E3A, F49A, A67T, A70T, and the like. The above notation means the amino acid before substitution, the position of substitution, and the amino acid after substitution in that order. For example, E3A means that glutamic acid (E), the amino acid corresponding to the third position based on the sequence of wild-type IGF-1, is substituted with alanine (A). The drug delivery system containing IGF-1 of the present invention can retain excellent blood-brain barrier permeability by including at least one amino acid substitution selected from the foregoing amino acid substitutions.

As used herein, the term "insulin-like growth factor-binding protein" or "IGFBP" serves as a transport protein for "insulin-like growth factor 1" or "IGF-1". Most IGFs in the body exist in a state of being bound to the IGFBP protein group, and thus inhibit the targeted delivery capability thereof. Therefore, the drug delivery system of the present invention may be designed not to bind to IGFBP, thereby increasing the BBB penetration efficiency.

In a specific aspect of the present invention, there may be provided a drug delivery system that has a structure of Fc-IGF1 in which an IGF-1 is linked to an immunoglobulin Fc region.

Herein, the term "Fc-IGF1 drug delivery system" may be used interchangeably with "Fc-IGF1 hybrid". The Fc-IGF1 drug delivery system not only exhibits excellent blood-brain barrier penetration efficiency by containing an IGF-1, which has inhibited binding to IGFBP and improved blood-brain barrier permeability, but also shows an increased half-life of a drug through the binding to an immunoglobulin Fc region.

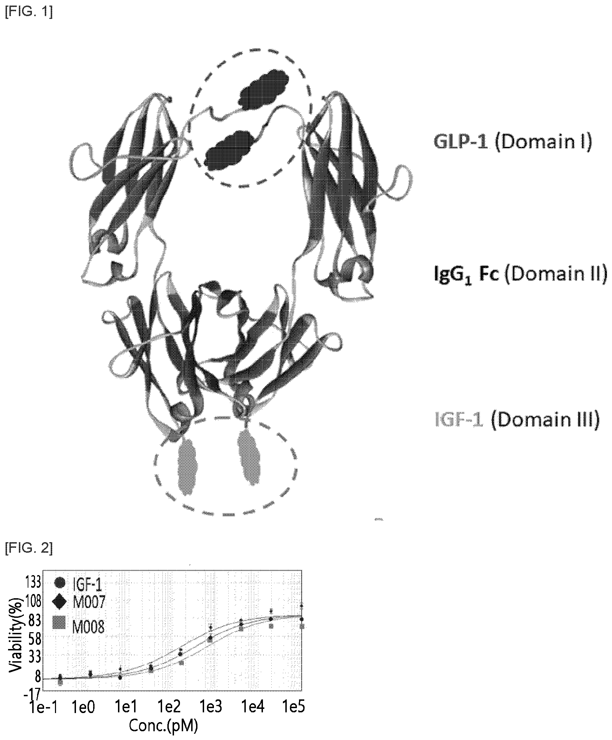

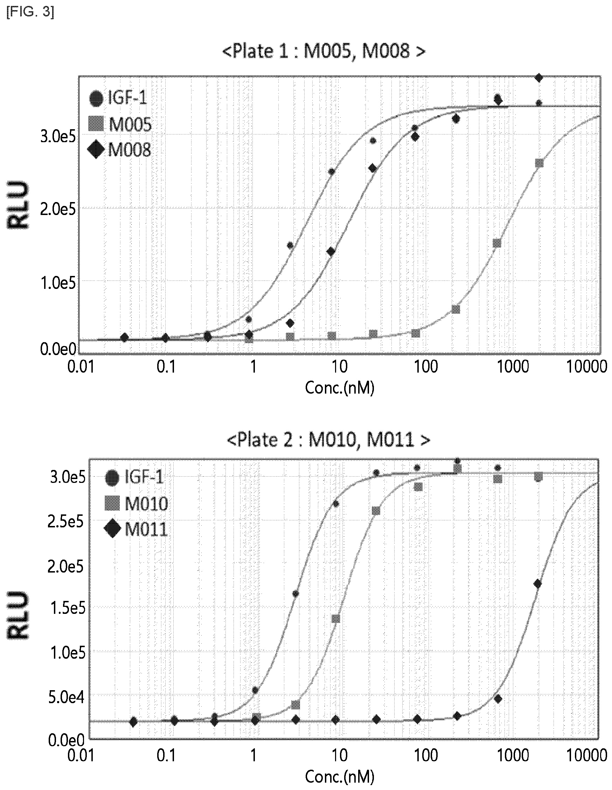

In an embodiment of the present invention, as a result of using penetration assay and PathHunter HEK293 IGF-1R bioassay kit in Saos-2 cell line to evaluate the IGF-1 activity of a fusion protein containing a GLP-1, an immunoglobulin Fc region, and an IGF-1, the fusion protein showed excellent IGF-1 activity compared with a control (wild) (FIG. 2 and FIG. 3).

As used herein, the term "immunoglobulin Fc" refers to a generic term for proteins that play an important role in immunity among serum components and act as antibodies. The basic structure thereof is composed of one pair of L-chain (light chain) of about 23,000 in molecular weight and one pair of H-chain (heavy chain) of 50,000 to 70,000 in molecular, linked weight by S-S bridging. These chains are classified as IgG, IgA, IgM, IgD, or IgE according to the type of H-chain. The immunoglobulin Fc region has a Y-shaped molecule, wherein the two top ends correspond to equivalent antigen-binding sites, while the bottom end (Fc fragment) is a site that shows biological activity that the antibody binding to antigen shows biological activity, such as binding to a complement or cell. Herein, "immunoglobulin Fc" may be used interchangeably with "immunoglobulin Fc region".

In the present invention, the immunoglobulin Fc fragment may be composed of one to four domains selected from the group consisting of CH1, CH2, CH3, and CH4 domains, and the immunoglobulin Fc fragment may further include a hinge region. The immunoglobulin Fc fragment may be selected from the group consisting of IgG, IgA, IgD, IgE, IgM, a combination thereof, and a hybrid thereof. A specific example of the immunoglobulin Fc region of the present invention may be trastuzumab, but is not limited thereto. The immunoglobulin Fc region may be mutated such that the drug delivery system of the present invention containing Fc has a reduced effector function in the body and, for example, may include a N297A mutation.

As used herein, the term "immunoglobulin G" or "IgG" refers to one type of immunoglobulin, which has placental permeability. IgG is classified into four subclasses IgG1, IgG2, IgG3, and IgG4 depending on the upper part of the constant region of the H chain.

As used herein, the term "human IgG1 Fc" refers to one of the antibody types, accounting for 75% of the total amount of immunoglobulins in human serum. In the present invention, the Fc region of a human-derived antibody (human IgG1) was used, and the corresponding domain is a protein functional group that is used in various pharmaceuticals for the purpose of improving the water solubility and half-life of a substance. The immunoglobulin Fc region of the present invention includes not only the native form but also a derivative or mutant thereof. The derivative or mutant refers to one in which at least one amino acid is mutated by substitution, deletion, addition, or the like, and retains its original activity. The amino acid sequence of human IgG1 Fc may include, but not limited to, the amino acid sequence of SEQ ID NO: 4 or may include a sequence in which a mutation, such as a substitution, a deletion, or an addition occurs at the amino acid corresponding to at least one of positions 297, 309, 311, 428, and a combination thereof in the amino acid sequence of SEQ ID NO: 4. Specifically, the amino acid corresponding to position 297 may be substituted with alanine, the amino acid corresponding to position 309 may be substituted with tyrosine, the amino acid corresponding to position 311 may be substituted with methionine, and the amino acid corresponding to position 428 may be substituted with leucine. More specifically, the amino acid sequence of SEQ ID NOS: 5, 6, 7, or 8 may be included. The amino acid sequence of SEQ ID NO: 4, 5, 6, 7, or 8 may include an amino acid sequence having at least 70%, 75%, 80%, 85%, 90%, 95%, 96%, 97%, 98%, 99%, 99.5%, 99.7%, or 99.9% homology or identity to the amino acid sequence set forth in SEQ ID NO: 4, 5, 6, 7, or 8. The "homology" and "identity" are as described above.

In the present invention, the human IgG1 Fc has functions of increasing the water solubility and in vivo half-life of a carrier, and thus enhance the preventive or therapeutic effects on neurological diseases.

In the present invention, FcRn refers to a protein that binds to the Fc region of an IgG antibody. FcRn may be derived from any organism including, but not limited to humans, mice, rats, rabbits, and monkeys. The in vivo half-life of immunoglobulins (antibodies) has been reported to be mediated by the binding of Fc to FcRn, and the half-life and persistence of antibodies in the blood largely depend on the binding between the Fc region of the antibodies and neonatal Fc receptor (FcRn), one of the IgG binding ligands. Additionally, the binding of Fc to FcRn also plays an important role in antibody delivery.

In an embodiment of the present invention, a fusion protein containing a GLP-1, an immunoglobulin Fc region, and an IGF-1 exhibited excellent binding affinity to FcRn (FIG. 6).

The drug delivery system of the present invention has a high blood-brain barrier penetration efficiency, so that the combination of a drug having the therapeutic activity with the drug delivery system having an Fc-IGF1 structure can ensure appropriate efficacy of the drug as well as long-lasting efficacy due to an increased half-life, leading to excellent therapeutic effects.

Specifically, a substance for the prevention or treatment of neurological diseases, which can be expected to exhibit excellent therapeutic effects by delivering the drug to penetrate the blood-brain barrier, may be linked to the drug delivery system of the present invention, but any substance that penetrates the blood-brain barrier to exhibit therapeutic effects may be linked to the drug delivery system of the present invention without limitation to a therapeutic agent for a specific disease.

The substance may be a therapeutic agent for neurological diseases, and the therapeutic agent for neurological diseases may be, for example, a small molecule drug, a peptide, an enzyme, an antibody, or the like for the treatment of neurological diseases. Examples of the therapeutic agent for neurological diseases may include celecoxib, masitinib, (1-3) IGF1, Exenatide BDNF, GDNF, CNTF, iduronate 2-sulfatase (IDS), glucocerebrosidase (GBA), α-synuclein specific antibody, and the like, and a specific example thereof may be a GLP-1. The drug delivery system Fc-IGF1 of the present invention may be in the form of a fusion protein linked to a GLP-1, but is not limited thereto.

As used herein, the term "GLP-1" refers to a type of gastrointestinal hormone derived from a transcript of a glucagon gene. The GLP-1 of the present invention includes not only the native form but also a derivative or mutant thereof. A derivative or mutant refers to one in which at least one amino acid is mutated by substitution, deletion, addition, or the like, and retains its original activity. The amino acid sequence of GLP-1 may include, but is not particularly limited to, any one of SEQ ID NOs: 1 to 3. The GLP-1 of the present invention may include the native sequence (SEQ ID NO: 2), include SEQ ID NO: 3 corresponding to amino acids 7-36, which is an active form thereof, or a GLP-1 mutant further including a substitution, addition, deletion, or the like of at least one amino acid in these sequences.

Alternatively, the GLP-1 may be mutated to have resistance to DPPIV in the native sequence, thus inhibiting its degradation in the body. As an example of mutation, the second amino acid alanine may be substituted with another amino acid (e.g., glycine (G)) or a non-native amino acid, but is not limited thereto.

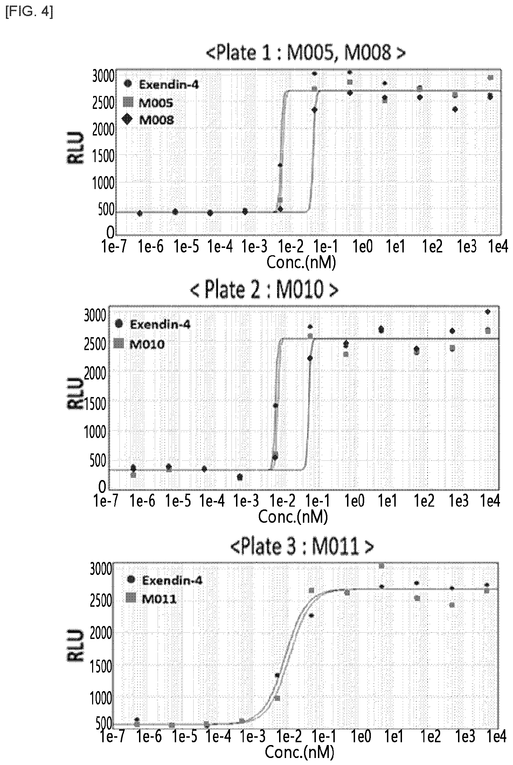

In an embodiment of the present invention, as a result of using the cAMP HunterTM Liraglutide bioassay kit to evaluate the GLP-1 activity of a fusion protein of the present invention, the fusion protein exhibited activity at a similar level to exendin-4, which is a GLP-1 receptor agonist (FIG. 4). In the present invention, the GLP-1 has functions as an anti-inflammatory action of a carrier in neurons, thus enhancing the preventive or therapeutic effects on neurological diseases.

In the present invention, a fusion protein containing a GLP-1, an immunoglobulin Fc region, and an IGF-1 is named PF1802.

In addition, the drug delivery system of the present invention may be linked to a therapeutic agent for amyotrophic lateral sclerosis (ALS). The therapeutic agent for amyotrophic lateral sclerosis may be, for example, a small-molecule drug, a peptide, an enzyme, an antibody, a protein, or the like for the treatment of amyotrophic lateral sclerosis. Examples of the therapeutic agent for amyotrophic lateral sclerosis may include celecoxib, masitinib, (1-3)IGF1, exenatide, BDNF, GDNF, CNTF, iduronate 2-sulfatase (IDS), glucocerebrosidase (GBA), α-synuclein specific antibody, and the like, and a specific example thereof may be a GLP-1, but is not limited thereto.

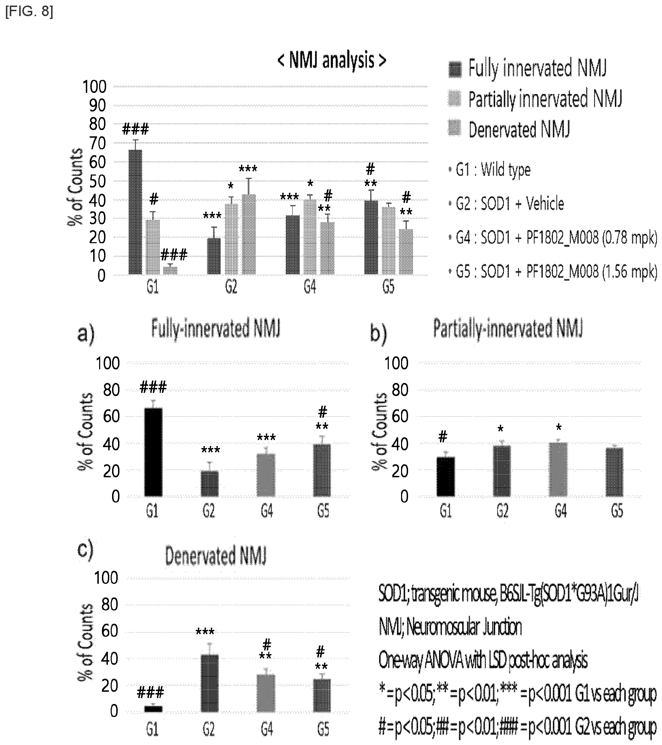

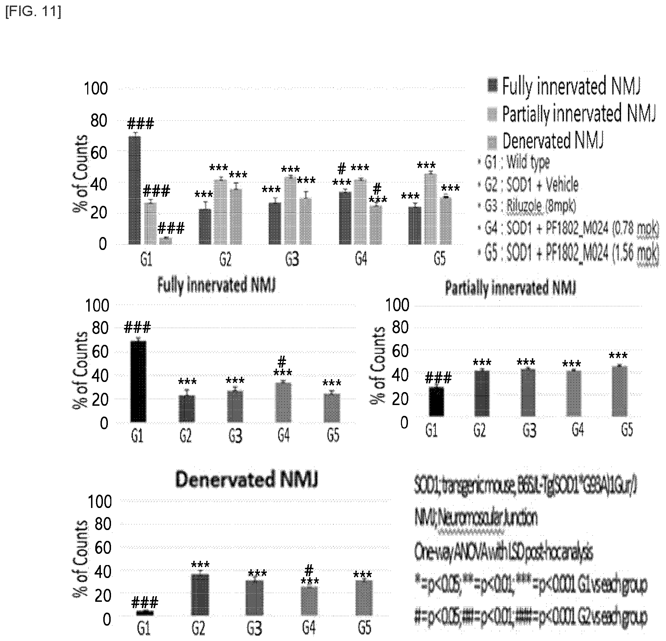

In an embodiment of the present invention, as a result of administering the PF1802_M008 candidate substance of the present invention to transgenic mouse models with amyotrophic lateral sclerosis, there was an increase in fully innervated neuromuscular junctions (NMJs) and a decrease in denervated neuromuscular junctions (NMJs). Thus, the candidate substance of the present invention was confirmed to exhibit excellent preventive and progression delay effects on amyotrophic lateral sclerosis disease (FIG. 8 and FIG. 11).

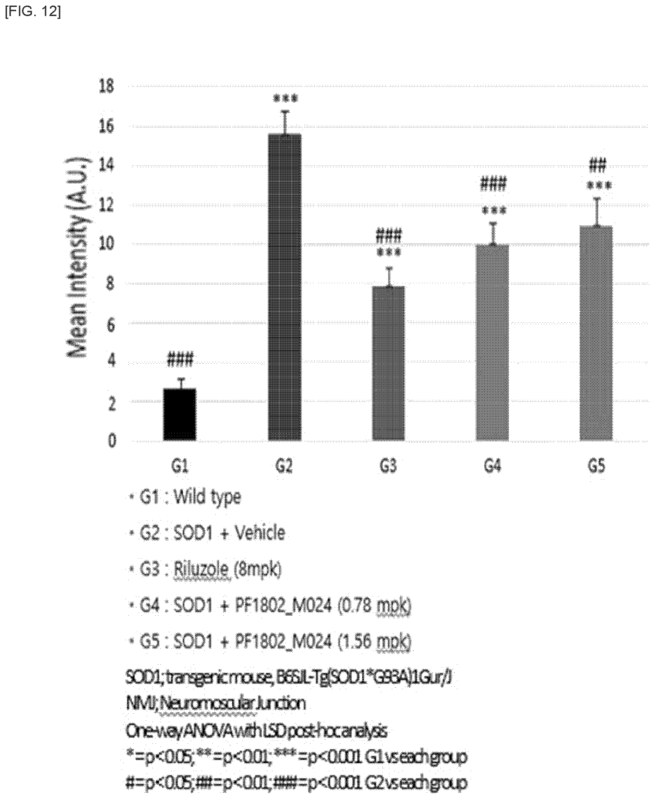

Additionally, a substance of the present invention exhibited a significant decrease in the microglial marker Iba1 compared with non-treatment and similar activity to riluzole known as an ALS therapeutic agent, confirming excellent BBB penetration (FIG. 12).

The drug delivery system of the present invention and a drug may be linked by various methods known in the art. Examples of the linking may include both linking the drug to an IGF-1 or Fc via a linker or directly linking therebetween via covalent or non-covalent linkage, and the linkage is not limited to a specific linking method or a finally linked form, as long as the linkage allows blood-brain barrier penetration and therapeutic effects.

The fusion protein of the present invention may have a form in which a GLP-1, a first linker, an Fc region, a second linker, and an IGF-1 are linked from the N-terminus.

The linkers each are a peptide linker, and the drug delivery system of the present invention may be one in which the IGF-1 and the immunoglobulin Fc region are fused through peptide linkers. One end of the linker may be linked to one chain of the immunoglobulin Fc region of a dimer, but is not limited thereto.

The peptide linker may contain one or more amino acids, for example, ranging from 1 to 1000 amino acids. The peptide linker may be any peptide linker known in the art, for example, including [GS]x linker, [GGGS]ₓ linker, and [GGGGS]ₓ linker, and the like, wherein x may be a natural number of 1 or greater (e.g., 1, 2, 3, 4, 5, or greater), but is not limited thereto.

Specifically, the linker of the present invention may be GSAPAP(G₄S) (SEQ ID NO: 38), (G₄S)₄GAHS (SEQ ID NO: 39), ASGAGSTTLEVLFQGP (SEQ ID NO: 40), (G)₈ (SEQ ID NO: 41), or (PA)₅ (SEQ ID NO: 42), but is not limited thereto.

In the present invention, the fusion protein may mean a structure that includes a GLP-1, an immunoglobulin Fc region, and an IGF-1, and may include any one of the amino acid sequences set forth in SEQ ID NOs: 25 to 37, but is not limited thereto. The amino acid sequences of SEQ ID NOs: 25 to 37 may include amino acid sequences having at least 70%, 75%, 80%, 85%, 90%, 95%, 96%, 97%, 98%, 99%, 99.5%, 99.7%, or 99.9% homology or identity to the amino acid sequence set forth in SEQ ID NO: 25 to 37. The "homology" and "identity" are as described above.

In accordance with another aspect of the present invention, there is provided a method for producing a drug delivery system, the method including linking an IGF-1 and an immunoglobulin Fc region.

In accordance with another aspect of the present invention, there is provided a method for producing a fusion protein, the method comprising linking a GLP-1, an immunoglobulin Fc region, and an IGF-1.

The "GLP-1", "IGF-1", "Fc", and "drug delivery system" are as described above.

In accordance with another aspect of the present invention, there is provided a pharmaceutical composition for preventing or treating a neurological disease, the pharmaceutical composition containing as an active ingredient a fusion protein containing a GLP-1, an immunoglobulin Fc region, and an IGF-1.

The "GLP-1", "immunoglobulin Fc" region, "IGF-1", and "fusion protein" are as described above.

The pharmaceutical composition may contain the fusion protein at a pharmaceutically effective amount.

The drug delivery system or fusion protein of the present invention has excellent blood-brain barrier (BBB) penetration efficiency, so that the efficacy of a therapeutic agent for a neurological disease, delivered to the brain through the drug delivery system or fusion protein, can be expected.

As used herein, the term "neurological disease" refers to a disease that causes a problem in the nervous system, and may include a neurological brain disease, a neurological muscle disease, and the like, and may specifically include neuromuscular junction disease, but is not limited thereto.

As used herein, the term "neurological brain disease" corresponds to brain tumors (glioma, meningioma, schwannoma, neurofibroma, pituitary adenoma, craniopharyngioma, metastatic brain tumor), cerebral infarction, hypertensive cerebral hemorrhage, subarachnoid hemorrhage, subdural hemorrhage, brain contusion, cerebral arteriovenous malformation, brain abscess, encephalitis, meningitis, hydrocephalus, epilepsy, arachnoid cyst, concussion, cerebral palsy, hemifacial spasm, Parkinson's disease, moyamoya disease, migraine, dementia, and the like.

As used herein, the term "neurological muscle disease" refers to a disease encompassing indirect muscular dysfunction caused by direct muscular diseases or abnormal nerves or neuromuscular junctions and is also called a neuromuscular disorder. If the central nervous system is affected, muscle spasms and paralysis occur, and different symptoms appear depending on the affected regions of the brain. Examples thereof may include stroke, multiple sclerosis, Parkinson's disease, peripheral neuropathy, myopathy, myasthenia gravis, amyotrophic lateral sclerosis, and spinal muscular atrophy.

The pharmaceutical composition of the present invention may have preventive or therapeutic effects on neurobiological diseases by exhibiting an improvement in neuromuscular junction function, a neuron protection effect, an improvement in blood-brain barrier permeability, and an increase in the half-life in the body, but is not limited thereto. **The** neurological diseases of the present invention are associated with the dysfunction of neuromuscular junctions, and the administration of the fusion protein according to the present invention can produce preventive or therapeutic effects on neurological diseases through the improvement and restoration of the functions of neuromuscular junctions.

As used herein, the term "prevention" refers to any action that inhibits or delays a neurological disease by administration of the composition of the present invention, and the term "treatment" refers to any action that alleviates or favorably changes the symptoms of a neurological disease by administration of the composition.

The pharmaceutical composition of the present invention may further contain a pharmaceutically acceptable carrier, excipient, or diluent. The pharmaceutically acceptable carrier, excipient, or diluent may be non-naturally occurring. Specifically, the composition may be formulated in the form of: an oral formulation, such as a powder, granules, a tablet, a capsule, a suspension, an emulsion, a syrup, or an aerosol; an externally-applied preparation; a suppository; and a sterile injectable solution, according to common methods, respectively. Examples of the carrier, excipient, and diluent that may be contained in the pharmaceutical composition of the present invention may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oils. The pharmaceutical composition may be prepared by using a diluent or excipient that is usually used, such as a filler, an extender, a binder, a humectant, a disintegrant, or a surfactant. Exemplary solid preparations for oral administration may include tablets, pills, powders, granules, capsules, and the like, and such solid preparations may be prepared by mixing the extract or fractions thereof with at least one excipient, for example, starch, calcium carbonate, sucrose or lactose, gelatin, and the like. Alternatively, lubricants, such as magnesium stearate and talc, may be used in addition to simple excipients. Liquid preparations for oral administration correspond to a suspension, a solution preparation for internal use, an emulsion, a syrup, and the like, and may contain not only frequently used simple diluents, such as water and liquid paraffin, but also several types of excipients, for example, a humectant, a sweetener, a flavor, and a preservative. Examples of preparations for parenteral administration include a sterile aqueous solution, a non-aqueous solvent, a suspension, an emulsion, a lyophilized preparation, and a suppository. Examples of the non-aqueous solvent and suspension may include propylene glycol, polyethylene glycol, a vegetable oil such as olive oil, an injectable ester such as ethylolate, and the like. Examples of a substrate for the suppository may include Witepsol, macrogol, Tween 61, cacao butter, laurin butter, glycerogelatin, and the like.

In accordance with another aspect of the present invention, there is provided a method for preventing or treating a neurological disease, the method including administering to a subject the drug delivery system, the fusion protein, or the pharmaceutical composition containing the same.

As used herein, the term "subject" may refer to any animal including humans that has or develops a neurological disease in the present invention. The administration of the pharmaceutical composition of the present invention to the subject can lead to a preventive and therapeutic effect on the neurological disease.

The pharmaceutical composition of the present invention is administered at a pharmaceutically effective amount.

As used herein, the term "administration" refers to the introduction of the pharmaceutical composition of the present invention to a subject by any suitable method, and the pharmaceutical composition may be administered through any typical route as long as the pharmaceutical composition can reach a target tissue. The pharmaceutical composition may be administered intraperitoneally, intravenously, intramuscularly, subcutaneously, intradermally, orally, locally, or intranasally, but is not limited thereto.

As used herein, the term "pharmaceutically effective amount" refers to an amount that is sufficient to prevent or treat a neurological disease at a reasonable benefit/risk ratio applicable to medical uses, and the level of the effective dose may be determined depending on factors including the kind of disease, disease severity, age and sex of a subject, the activity of a drug, sensitivity to a drug, time of administration, route of administration, rate of excretion, duration of treatment, and drugs used in combination with the composition, and other factors well known in the medical field. For example, the drug delivery system, the fusion protein, or the pharmaceutical composition containing the same may be administered at a dose of 0.01 to 500 mg/kg, specifically, 10 to 100 mg/kg per day, and the administration may be performed once a day or divided into several times.

The composition of the present invention may be administered as an individual therapeutic agent or in combination with other therapeutic agents, and may be sequentially or simultaneously administered together with a conventional therapeutic agent. In addition, the composition may be administered once or multiple times. It is important to administer a minimal amount that is effective in a maximal extent without any adverse action, considering all the factors. The dosage may be determined by those skilled in this art.

The composition of the present invention may be used alone or may be used in combination with surgical operation, hormonal therapy, chemotherapy, and methods using biological response regulators to prevent or treat a neurological disease.

In the present invention, the composition may have effects of restoring or improving functions of neuromuscular junctions (NMJs).

As used herein, the term "neuromuscular junction" or "NMJ" refers to a type of synapsis with a specific structure in which the motor neuron connects the muscle at the end thereof and the nerve impulse is transmitted to the membrane of muscle fibers. Exemplary neuromuscular junction diseases include myasthenia gravis (MG), Lambert-Eaton myasthenic syndrome, botulism, and congenital myasthenic syndrome.

In accordance with another aspect of the present invention, there is provided a pharmaceutical composition for the prevention or treatment of amyotrophic lateral sclerosis (ALS), the pharmaceutical composition containing the drug delivery system or the fusion protein.

The "drug delivery system", "fusion protein", and "pharmaceutical composition" are as described above.

In the present invention, the fusion protein may be one in which a GLP-1 as a therapeutic agent may be linked to the drug delivery system of the present invention, and specifically, a GLP-1, an immunoglobulin Fc region, and an IGF-1 may be linked to the drug delivery system of the present invention. The fusion protein according to the present invention exhibits a high efficiency in reaching the brain and an increased half-life, and thus can be expected to have an excellent therapeutic effect on amyotrophic lateral sclerosis.

In accordance with another aspect of the present invention, there is provided use of a fusion protein containing a GLP-1, an immunoglobulin Fc region, and an IGF-1 for preventing, alleviating, or treating a neurological disease.

In accordance with another aspect of the present invention, there is provided use of a pharmaceutical composition containing the fusion protein for preventing or treating a neurological disease.

The "GLP-1", "immunoglobulin Fc region", "IGF-1", "neurological disease", and "pharmaceutical composition" are as described above.

### [Best Mode for Carrying Out the Invention]

Hereinafter, the present invention will be described in detail through examples. These examples are given for specifically illustrating the present invention, and the scope of the present invention is not limited thereto.

### Example 1: Preparation of fusion protein

For securing candidate substances, GLP1-Fc-IGF1 or GLP1-Fc-mut. IGF1-TfR was synthesized, and then cloned into pcDNA3.1 vectors using Nhe I/Hind III sites. The cloned vectors were transfected into CHO-S cells, and the supernatants were collected on the 5th day. Thereafter, cells and floaters were removed through centrifugation and a 0.22-µm filter. Afterwards, purification with protein A resin was performed to obtain various candidate substances PF1802 _M003 to M032 (SEQ ID NOs. 13 to 37) as shown in Table 1 (FIG. 1 and Table 1).

**TABLE 1**

| **Code** | **Feature** | **Linker 1** | **Fc** | **Linker 2** | **IGF1** | **SEQ ID NO** |
|---|---|---|---|---|---|---|
| M003 | GLP1-Fc-IGF1 | GSAPAP(G₄ S) | Original | (G4S)₄G AHS | WT | 13 |
| M004 | GLP1-Fc-mut. IGF1 | GSAPAP(G₄ S) | Original | (G4S)₄G AHS | A67T, A70T | 14 |
| M005 | GLP1-Fc-IGF1 | GSAPAP(G₄ S) | N297A | (G4S)₄G AHS | WT | 15 |
| M006 | GLP1-Fc-mut. IGF1 | GSAPAP(G₄ S) | N297A | (G4S)₄G AHS | A67T, A70T | 16 |
| M007 | GLP1-Fc-mut. IGF1 | GSAPAP(G₄ S) | N297A | (G4S)₄G AHS | E3A, F49A | 17 |
| M008 | GLP1-Fc-mut. IGF1 | GSAPAP(G₄ S) | N297A | (G4S)₄G AHS | A67T, A70T, E3A, F49A | 18 |
| M009 | GLP1-Fc-IGF1-TfR | GSAPAP(G₄ S) | N297A | (G4S)₄G AHS | WT | 19 |
| M010 | GLP1-Fc-mut. IGF1-TfR | GSAPAP(G₄ S) | N297A | (G4S)₄G AHS | A67T, A70T, E3A, F49A | 20 |
| M011 | GLP1-Fc | GSAPAP(G₄ S) | N297A | - | - | 21 |
| M012 | Fc-IGF1 | - | N297A | (G4S)₄G AHS | WT | 22 |
| M013 | Fc-mut.IGF1 | - | N297A | (G4S)₄G AHS | A67T, A70T, E3A, F49A | 23 |
| M016 | Fc-mut.IGF1-TfR | - | N297A | (G4S)₄G AHS | A67T, A70T, E3A, F49A | 24 |
| M020 | GLP1-Fc-mut.IGF1 | GSAPAP(G₄ S) | N297A, L309Y, Q311M, M428L | (G4S)₄G AHS | A67T, A70T, E3A, F49A | 25 |
| M021 | GLP1-Fc-mut.IGF1 | GSAPAP(G₄ S) | N297A, L309E, Q311M, M428L | (G4S)₄G AHS | A67T, A70T, E3A, F49A | 26 |
| M022 | GLP1-Fc-mut.IGF1 | GSAPAP(G₄ S) | N297A, L309E, Q311W, M428L | (G4S)₄G AHS | A67T, A70T, E3A, F49A | 27 |
| M023 | GLP1-Fc-mut.IGF1 | GSAPAP(G₄ S) | N297A, Lys-del | ASGAGS TTLEVLF QGP | A67T, A70T, E3A, F49A | 28 |
| M024 | GLP1-Fc-mut.IGF1 | GSAPAP(G₄ S) | N297A, Lys-del | (G)₈ | A67T, A70T, E3A, F49A | 29 |
| M025 | GLP1-Fc-mut.IGF1 | GSAPAP(G₄ S) | N297A, Lys-del | (PA)₅ | A67T, A70T, E3A, F49A | 30 |
| M026 | GLP1-Fc | GSAPAP(G₄ S) | N297A, I ys-del | - | A67T, A70T, E3A, F49A | 31 |
| M027 | Fc-mut.IGF1 | - | N297A, Lys-del | (G)₈ | A67T, A70T, E3A, F49A | 32 |
| M028 | Fc-mut.IGF1 | - | N297A, Lys-del | (PA)₅ | A67T, A70T, E3A, F49A | 33 |
| M029 | Fc-mut.IGF1 | - | N297A, Lys-del | - | A67T, A70T, E3A, F49A | 34 |
| M030 | GLP1-Fc-mut.IGF1 | GSAPAP(G₄ S) | N297A, L309Y, Q311M, M428L, Lys-del | (G)₈ | A67T, A70T, E3A, F49A | 35 |
| M031 | GLP1-Fc-mut.IGF1 | GSAPAP(G₄ S) | N297A, L309Y, Q311M, M428L, Lys-del | (G)₈ | A67T, A70T, E3A, F49A | 36 |
| M032 | GLP1-Fc-mut.IGF1 | GSAPAP(G₄ S) | N297A, L309Y, Q311M, M428L, Lys-del | (G)₈ | A67T, A70T, E3A, F49A | 37 |

| | | | | | | |
|---|---|---|---|---|---|---|
| * TfR: Transferrin receptor binding peptide | | | | | | |

### Example 2: Evaluation of activity of PF1802 candidate substances

### Example 2-1. Evaluation of IGF-1 activity of PF1802 candidate substances using Saos-2 cell line

To investigate the IGF-1 activity of the PF1802 candidate substances prepared by the method of Example 1, a proliferation assay by IGF-1 was performed on the Saos-2 cell line. The Saos-2 cell line was dispensed into a 96-well plate at 1×10⁴ per well, incubated at 37°C and 5% CO₂ for 16 hours, and subjected to starvation for 4 hours. Afterwards, the PF1802 candidate substances were diluted 5-fold from a maximum concentration of 100 nM to 0.00256 nM, added to each well, and then incubated at 37°C and 5% CO₂ for 48 hours. The incubated cells were stained with sulforhodamine B (SRB), and then the absorbance was measured at 540 nm using a microplate reader. The PLA statics function was applied to establish IGF-1 as the reference, and then the relative potency values of the PF1802 candidate substances were calculated (Table 2 and FIG. 2).

**TABLE 2**

| **Sample** | **Rel. Pot. (%)** |
|---|---|
| IGF-1 | 100.0 |
| M007 (GLP1-aglycosylated Fc-mut IGF1(3,49)) | **24.6** |
| M008 (GLP1-aplycosylated Fc-mut IGF1(3,49,67,70)) | **46.4** |

As a result, as shown in Table 2 above, the IGF-1 activity of the mutant IGF-1 substances M007 and M008 was confirmed by comparison with the substance using wt.IGF1

### Example 2-2. Evaluation of IGF-1 activity of PF1802 candidate substances

In order to investigate the IGF-1 activity of the PF1802 candidate substances prepared by the method of Example 1, the IGF-1 activity (phosphorylation activity) of the produced PF1802 substances was measured using the PathHunter HEK293 IGF-1R bioassay kit (Discover X, 95-0505Y1-000070), which was a kit using IGF1R-overexpressed cell line. Specifically, as shown in Table 3, the measurement was performed according to the technical manual of the assay kit, and the results were derived by application of a 4-parameter fit (Table 4 and FIG. 3).

**TABLE 3**

| **Day** | **Parameters** | **Preparation** | **Conditions** |
|---|---|---|---|
| 1 | Cell seeding | HEK 293 IGF-1R | 3 × 10⁴ cells/well37 °C, 5 % CO₂, 1h |
| | Sample | Positive control (IGF-1), PF1802 | 10 µL/well37 °C, 5 % CO₂, 16h |
| 2 | Reagent | Detection reagent 1 | 10 µL/well R/T, dark, 15 min |
| | | Detection reagent 2 | 40 µL/well R/T, dark, 1h |
| | Measurement | Plate reader | Chemiluminescent signal |

**TABLE 4**

| Sample | EC₅₀ (nM) |
|---|---|
| IGF-1 | 3.889 / 2.773 |
| M005 (GLP1-Fc- IGF1) | 780.8 |
| M008 (GLP1-Fc-mut IGF1) | 13.93 |
| M010 (GLP1-Fc-mut IGF1 -TfR) | 9.914 |
| M011 (Fc- GLP1 ) | > 2000 |

As a result, as shown in Table 4, the PF1802 candidate substances M005, M008, and M010 were confirmed to exhibit the IGF-1 activity when compared with M011 (negative control), which was an IGF1-free substance.

### Example 2-3. Evaluation of GLP-1 activity of PF1802 candidate substances

In order to investigate the GLP-1 activity of the PF1802 candidate substances prepared by the method of Example 1, the GLP-1 activity of the produced substances was measured using the cAMP Hunter^{™} Liraglutide bioassay kit (Discover X, 95-0062Y2-00100). Specifically, as shown in Table 5, the measurement was performed according to the technical manual of the assay kit, and the results were derived by applying a 4-parameter fit (Table 6 and FIG. 4).

**TABLE 5**

| **Day** | **Parameters** | **Preparation** | **Conditions** |
|---|---|---|---|
| 1 | Cell seeding | CHO-K1 GLP1R | 3 × 10⁴ **cells/well37** °C, 5 % CO₂, 24h |
| 2 | Sample | PF1802, Exendin-4(Positive control) | **15** µL/well37 °C, 5 % CO₂, 30 min |
| | Antibody | cAMP Ab reagent | 15 µL/well |
| | Cell lysis | cAMP Working detection solution | 60 µL/well R/T, dark, 1h |
| | Hydrolysis | cAMP solution A | 60 µL/well R/T, dark, 3h |
| | Measurement | Plate reader | Chemiluminescent signal |

**TABLE 6**

| Sample | EC₅₀ (pM) |
|---|---|
| Exendin-4 | 5.0 /5.0 /8.0 |
| M005 | 6.0 |
| M008 | 20.0 |
| M010 | 6.0 |
| M011 | 11.0 |

As a result, as shown in Table 6, the GLP-1 activity of M005, M008, M010, and M011 had a similar level to that of exendin-4, indicating that the GLP-1 activity of the produced substances were not problematic.

### Example 2-4. Evaluation of pharmacokinetics (PK) of PF1802 candidate substances

To investigate the pharmacokinetics of the PF1802 candidate substances in blood, the candidate substances were subcutaneously administered to mice at a dose of 5 mpk, and then blood collection was conducted for up to 144 hours. Thereafter, the PK parameter values were obtained using WinNolin software on the basis of ELISA results (Table 7 and FIG. 5).

**TABLE 7**

| | **M005** | **M010** |
|---|---|---|
| Cmax (ng/mL) | 7896.1 | 400925.2 |
| Tₘₐₓ **(hr)** | 48.0 | 16.0 |
| AUCₗₐₛₜ (hr·ng/mL) | 761136.2 | 12197889.5 |
| AUC_{inf} (hr·ng/mL) | 1003755.1 | 12327010.1 |
| T_{1/2} (hr) | 61.0 | 29.4 |
| CL/F (mL/hr/kg) | 5.0 | 0.4 |
| Vd/F (mL/Kg) | 438.3 | 17.2 |
| Rsq_adjusted | 0.7 | 1.0 |
| % AUCₑₓₚ (%) | 24.2 | 1.0 |

As a result, as shown in Table 7, the median Tmax values of the candidate substances M005 and M010 were 48 hours and 16 hours, respectively, indicating that the candidate substances were slowly absorbed, and the half-life values were 61 hours and 29.4 hours, respectively.

These results suggest that upon actual drug administration, the candidate substances of the present invention exhibit sustained efficacy and can shorten the interval of drug administration.

### Example 2-5. Evaluation of binding affinity of half-life-increasing Fc-introduced PF1802 candidate substances to human FcRn

To investigate the binding affinity of the PF1802_M020, M021, and M022 substances, obtained by introducing three types of blood half-life-increasing Fc to the PF1802-M008 candidate substance, to human FcRn, ELISA was performed, and to investigate the difference in binding affinity depending on pH, two situations at pH 6.0 and pH 7.4 were prepared, respectively. Specifically, each of the PF1802_M020, M021, and M022 substances was coated at 4 µg/mL on a 96 well immunoplate, and reacted with PBS (pH 6.0 and 7.4) containing 4% skim milk at room temperature for 1 hour to block non-specific binding. Thereafter, the Human FcRn-GST protein was diluted 4-fold from a maximum concentration of 5 ug/mL to 0.0003 µg/mL using PBS (pH 6.0, 7.4) containing 1% skim milk, and then dispensed into each well, followed by incubation at room temperature for 1 hour. Afterwards, the wells were treated with anti-GST Ab-HRP (cytiva, 27457701), followed by treatment with TMB solution and H₂SO₄, and the absorbance was measured at 450 nm to obtain the results (FIG. 6).

As a result, the blood half-life-increasing Fc-introduced PF1802_M020, M021, and M022 candidate substances showed significant an increase in binding affinity to human FcRn compared with PF1802_M008. These results indicate excellent binding affinity to human FcRn at pH 6.0, which is important in terms of blood half-life increase.

Furthermore, PF1802_M030, M031, and M032 candidate substances (SEQ ID NO: 35, 36, and 37) were ensured by introducing the same three types of blood half-life-increasing Fc as above into the PF1802_M024 candidate substance, for which substance optimization was completed through Examples 4 and 5 to be later described, and the PF1802_M030, M031, and M032 candidate substances also showed an increase in binding affinity and excellent binding affinity to Human FcRn.

### Example 3: Evaluation of in vivo efficacy of PF1802 M008 candidate substance

### Example 3-1. Evaluation of mouse brain blood barrier (BBB) permeability of PF1802 M008 candidate substance

To investigate BBB permeability, IVISense 680 NHS Fluorescent labeling kit (PerkinElmer) was used, and the IGF-1-free M011 (GLP1-Fc) candidate substance was used as a negative control. Specifically, a fluorescently labeled substance was intraperitoneally administered once to a mouse (Balb/C male) and then perfused with physiological saline at 24 and 72 hours. Then, the brain of mice was extracted and subjected to imaging analysis using a secondary optical imaging device (IVIS spectrum) (FIG. 7).

As a result, the brain tissue fluorescence intensity was in the order M008 > M005 > M011. The fluorescence intensity of M005 was 1.1 times (at 24 hours) higher than that of M011 and 1.2 times higher (at 72 hours), with no statistically significant difference. The fluorescence intensity of M008 was 1.7 times (at 24 hours) higher than that of M011 and 2.6 times higher (at 72 hours), showing the highest fluorescence intensity, with statistical significance.

These results indicate that the BBB permeability of the PF1802_M008 candidate substance was excellent and the BBB permeability of mut.IGF1 was significantly higher than that of wt.IGF1.

### Example 3-2. Assay of efficacy of PF1802 M008 candidate substance in amyotrophic lateral sclerosis (ALS) transgenic mouse models

To investigate the efficacy of the PF1802_M008 candidate substance, the PF1802_M008 candidate substance was administered subcutaneously to 9-week-old SOD-1 (G39A) mice twice a week for 10 weeks, as shown in Table 8. The mice were sacrificed at 109 days of age for immunohistological analysis, and for evaluation of neuromuscular junctions (NMJs), gastrocnemius muscle samples were stained with anti-synatotagmin 2 Ab and alpha-bungarotoxin, and then subjected to imaging analysis using a fluorescence microscope (FIG. 8).

**TABLE 8**

| Group | Number of animals | Type of mouse | Substance administered | Method of administratio n | Period of administratio n | Concentration of administration |
|---|---|---|---|---|---|---|
| G1 | 6 | WT | - | - | - | - |
| G2 | 6 | SOD1 | Vehicle | Subcutaneou s | 2QW x 10 | - |
| G4 | 6 | SOD1 | PF1802_M008 | Subcutaneou s | 2QW × 10 | 0.78 mg/kg |
| G5 | 6 | SOD1 | PF1802_M008 | Subcutaneou s | 2QW × 10 | 1.56 mg/kg |

As a result, G4 and G5 groups administered the PF1802_M008 candidate substance showed an increase in fully innervated neuromuscular junctions (NMJs) in a dose-dependent manner, and a decrease in denervated neuromuscular junctions (NMJs).

These results indicate that the treatment with the PF1802_M008 candidate substance exhibits preventive and progression delay effects on amyotrophic lateral sclerosis (ALS) disease.

### Example 4: Optimization of PF1802 candidate substances

### Example 4-1. Structural analysis of PF1802 M008 candidate substance and preparation of optimized substance

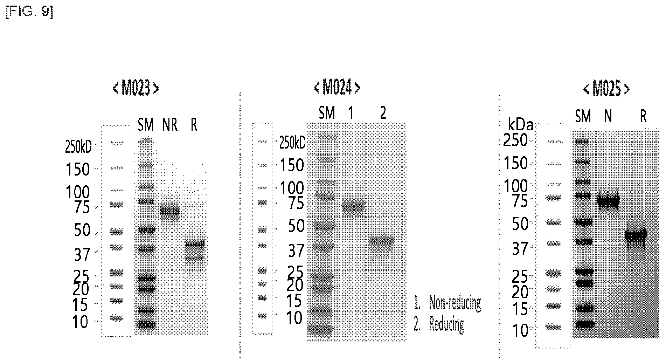

For optimization of the PF1802_M008 candidate substance, of which the efficacy had been confirmed through Example 2 and Example 3, M023, M024, and M025 candidate substances were designed by introducing three different types of linkers thereto, respectively. The substances were prepared by the same method as in Example 1. Then, the prepared M023, M024, and M025 candidate substances were subjected to SDS-PAGE gel analysis under non-reducing and reducing conditions. As a result, the M024 and M025 substances were not problematic except for M023 confirmed to be in a cleavage form. (FIG. 9).

### Example 4-2. Evaluation of binding affinity of PF1802 M024, and M025 candidate substances using SPR

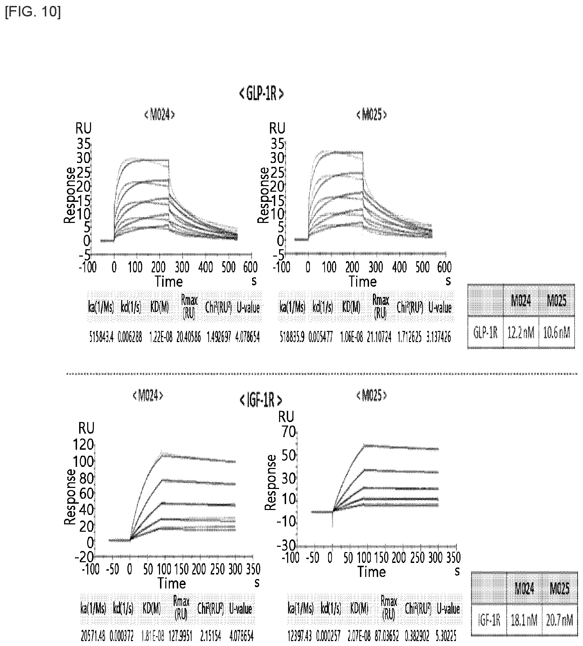

To investigate the binding affinity of the optimized PF1802_M024 and M025 prepared in Example 4-1 to GLP-1R and IGF-1R, signal changes resulting from binding were measured using SPR. Specifically, a CM5 sensor chip was mounted on BIAcore T200 (GE Healthcare), and then 100 mM N-hydroxysuccinimide (NHS) and 400 mM 1-ethyl-3-(3-dimethylaminopropyl)carbodiimide (EDC) were injected at a 1:1 ratio to activate flow cells, followed by the immobilization of anti-His tag antibody at a level of 8000 to 10000 RU. GLP-1R-His and IGF-1R-His recombinant proteins used as ligands were each injected at a concentration of 2.5 ug/mL for each cycle and captured at a level of 280 to 290 RU. Afterwards, the M024 and M025 candidate substances were injected under the conditions in Table 9, followed by the measurement of SPR binding affinity (FIG. 10).

**TABLE 9**

| **Ligand** | **M024 and M025 concentrations** | **Injection conditions** |
|---|---|---|
| GLP-1R | 125.0 ~ 3.9 nM (2-fold serial dilution) | Association : 240 sec |
| | | Dissociation : 300 sec |
| | | Flow rate : 30 uL/min |
| IGF-1R | 1000.0 ~ 31.3 nM (2-fold serial dilution) | Association : 90 sec |
| | | Dissociation : 210 sec |
| | | Flow rate : 30 uL/min |

As a result, the SPR binding affinity of the M024 candidate substance was 12.2 nM (GLP-1R) and 18.1 nM (IGF-1R), and the SPR binding affinity of the M025 candidate substance was 10.6 nM (GLP-1R) and 20.7 nM (IGF-1R).

These results indicate that both the M024 and M025 candidate substances bind to GLP-1R and IGF-1R and there was no difference in binding affinity between the two candidate substances, with no significant difference. This suggests that the M024 and M025 candidate substances exhibit excellent GLP-1 and IGF-1 activities.

### Example 5: Assay of efficacy of PF1802 M024 candidate substance

### Example 5-1. Assay of efficacy of PF1802 M024 candidate substance in ALS transgenic mouse models

To investigate the efficacy of the PF1802_M024 candidate substance, of which the optimization had been completed through Example 4, the PF1802_M024 candidate substance was administered subcutaneously to 10-week-old SOD-1(G39A) mice twice a week for 8 weeks, as shown in Table 10. The mice were sacrificed at 115 days of age for immunohistological analysis. For evaluation of neuromuscular junctions, gastrocnemius muscle samples were stained with anti-synatotagmin 2 Ab and alpha-bungarotoxin (FIG. 11), and for checking of nerve inflammation, spinal cord samples were stained with anti-lba1 Ab (FIG. 12) and then subjected to imaging analysis using a fluorescence microscope.

**TABLE 10**

| Group | Number of animals | Type of mouse | Substance administered | Method of administration | Period of administratio n | Concentration of administration |
|---|---|---|---|---|---|---|
| G1 | 12 | WT | - | - | - | - |
| G2 | 9 | SOD1 | Vehicle | Subcutaneous | 2QW × 8 | - |
| G3 | 12 | SOD1 | Riluzole | Oral gavage | QD | 8.00 mg/kg |
| G4 | 12 | SOD1 | PF1802_M002 4 | Subcutaneous | 2QW x 8 | 0.78 mg/kg |
| G5 | 12 | SOD1 | PF1802_M002 4 | Subcutaneous | 2QW x 8 | 1.56 mg/kg |

As a result, G4 and G5 groups administered the PF1802_M024 candidate substance showed an increase in fully innervated neuromuscular junctions (NMJs) and a decrease in denervated neuromuscular junctions (NMJs). Additionally, G4 and G5 groups showed a significant reduction in Iba1, which enables the confirmation of inflammation presence or absence, compared with non-treatment, and showed activity at a similar level to that of a positive control.

The results of NMJ improvement and Iba1 reduction indicate that the PF1802_M024 candidate substance has preventive and delaying effects on amyotrophic lateral sclerosis disease, and the results of Iba1 reduction in the spinal cord samples validate that the PF1802_M024 candidate substance penetrates BBB.

### Example 5-2. Evaluation of mouse BBB permeability of PF1802 M024 candidate substance

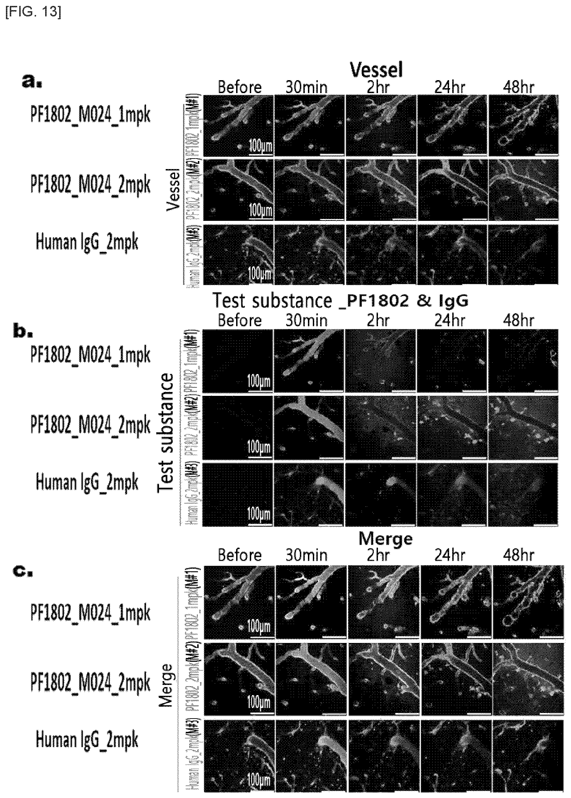

To investigate BBB permeability, a substance fluorescently labeled by using an Alexa fluor 647 antibody labeling kit (PerkinElmer) was intravenously administered once to cranial imaging window mouse models (C57BL/6N) and then imaged over time. The brain vessel sites were marked in red (FIG. 13a), and the test substance (PF1802 & Human IgG) was marked in green (FIG. 13b). The cortex was marked in purple, which can be observed in merged images of the brain vessel and the test substance (FIG. 13c).

The results indicate that the PF1802_M024 candidate substance showed excellent BBB permeability compared with human IgG.

While the present invention has been described with reference to particular illustrative embodiments, a person skilled in the art to which the present invention pertains can understand that the present invention may be embodied in other specific forms without departing from the technical spirit or essential characteristics thereof. Therefore, the embodiments described above should be construed as being exemplified and not limiting the present invention. The scope of the invention should be construed based on the meaning and scope of the appended claims, rather than the detailed description, and all changes or variations derived from equivalent concepts fall within the scope of the present invention.

## Claims

1. A fusion protein comprising a GLP-1, an immunoglobulin Fc region, and an IGF-1.

2. The fusion protein of claim 1, wherein the immunoglobulin Fc fragment is selected from the group consisting of IgG, IgA, IgD, IgE, IgM, combinations thereof, and hybrids thereof.

3. The fusion protein of claim 1, wherein the immunoglobulin Fc fragment is derived from IgG1.

4. A pharmaceutical composition for the prevention or treatment of a neurological disease, the pharmaceutical composition comprising as an active ingredient a fusion protein containing a GLP-1, an immunoglobulin Fc region, and an IGF-1.

5. The pharmaceutical composition of claim 4, wherein the neurological disease is any one selected from the group consisting of Parkinson's disease, Alzheimer's dementia (senile dementia), stroke, Lou Gehrig's disease, Pick's disease, Creutzfeldt-Jakob disease, Huntington's disease, progressive supranuclear palsy, spinocerebellar degeneration, cerebellar atrophy, multiple sclerosis, amyotrophic lateral sclerosis, peripheral neuropathy, myasthenia gravis, and spinal muscular atrophy.

6. The pharmaceutical composition of claim 4, wherein the pharmaceutical composition has an effect of restoring or improving a function of neuromuscular junctions (NMJs).

7. A method for preventing or treating a neurological disease, the method comprising administering the composition of claim 4 to a subject.
